# EUROPEAN PATENT APPLICATION

(11) **EP 3 199 522 A1**
(43) Date of publication of application: **02.08.2017**
(21) Application number: 16153618.0
(22) Date of filing: 01.02.2016
(51) Int. Cl.: C07D 211/02, C07C 327/22, C07D 211/90, C07D 213/127

(54) **AMINOTHIOATE DERIVATIVES, PRODUCTION THEREOF AND USE**

(71) Applicant: Solvay SA, 1120 Bruxelles (BE)
(72) Inventor: BRAUN, Max Josef, Wedemark (DE)
(74) Representative: Mross, Stefan P.M.

(57) **Abstract**

The present invention concerns aminothioate derivatives of formula (I) 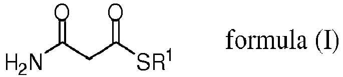 methods for production and use thereof.

## Description

The present invention concerns aminothioate derivatives, methods for production and use thereof.

Malonic monoamide derivatives are known to react with alkenones to form the respective dihydropyridinecarboxylates, which are useful intermediates in the synthesis of agrochemicals and pharmaceuticals. The manufacture of dihydropyridinecarboxylates from malonic monoamide derivatives is described, for example, in WO2010/037688. They can further be converted into pyridines and related compounds. Alkylthiopyridines are known, for example from US2008003180, to be valuable intermediates for the manufacture of agrochemical compounds.

The present invention concerns new compounds of the formula **(I)**

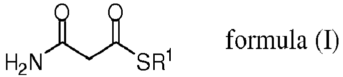

The invention also concerns a process for the manufacture of the compounds of formula **(I).**

The invention further concerns the use of the compounds of formula **(I)** in the manufacture of compound of formula **(II)**

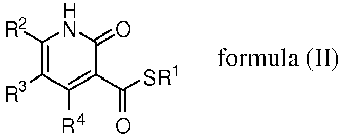

The compounds according to the present invention allow for efficient synthesis of dihydropyridinecarbothioates, which are useful intermediates for the preparation of pyridines. Such pyridines are precursors of, for example, agrochemicals and pharmaceuticals.

The term "alkyl" intends straight and branched alkyl residues.

In the present invention, R¹ in the compound of formula (I) is selected from the group consisting of H and optionally substituted C₁-C₄-alkyl. Preferably, R¹ is methyl or ethyl. Most preferably, R¹ is methyl. R¹ is optionally substituted with one or more substituents independently selected from the group consisting of R', X', OR', SR', CN, C(O)R', COOR', C(O)NR'₂, wherein R' is selected from the group consisting of hydrogen, C₁-C₁₂-alkyl group, CN, C₂-C₆ alkenyl, aryl, cycloalkyl, aralkyl, heteroaryl, each of which is optionally substituted by one or more groups independently selected from the group consisting of -R", -X', -OR", -SR", -NR"₂, -SiR'₃, -COOR', -(C-O)R", -CN and -CONR"₂, wherein R" is hydrogen or a C₁-C₁₂-alkyl group and X' is F, Cl, Br, or I. Preferably, R¹ is CH₃.

The invention concerns further a process for the manufacture of the compound of formula (I). The process comprises at least one of the following steps :
a) reacting malonic acid dichloride with a compound of formula MSR¹, wherein R¹ is defined as before, and wherein M is a monovalent alkali metal ion, preferably Na⁺, to obtain the compound of formula (III) 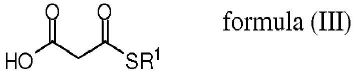
b) reacting the compound of formula (III) with a chlorinating agent to obtain a compound of formula (IV) 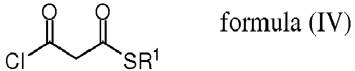
c) reacting a malonic ester of the formula (IX) R⁸OC(O)CH₂C(O)OR⁸ with a compound of the formula R¹SH, wherein R¹ is defined as before, to obtain a compound of the formula (VIII) 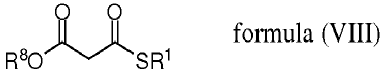 wherein R⁸ is a C₁-C₁₂-alkyl group, preferably methyl or ethyl.
d) reacting the compound of formula (IV) or (VIII) with ammonia or an ammonia derivative to obtain a compound of formula (I).

An ammonia derivative is, for example, NH₄OH.

The chlorinating agent of step b) often is selected from (COCl)₂ and SOCl₂.

The process for the manufacture of the compound of formula (I) can comprise at least one step of obtaining a fraction enriched in compound (III), (IV) or (I). This at least one step is selected from distillation, chromatography, treating with aqueous or organic additives, extracting, filtration, drying and crystallization.

The process for the manufacture of compound (I) preferably is performed continuously. In a preferred embodiment, at least one of the step comprised in the process for the manufacture of compound (I) is carried out in a microreactor, and/or under turbulent conditions.

The invention further concerns the use of the compound of formula (I) for the manufacture of the compound of formula (V) 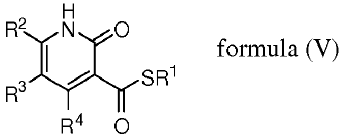 wherein R¹ is as defined above, and R², R³ and R⁴ are independently selected from the group consisting of R', X', OR', SR', CN, C(O)R', COOR', C(O)NR'₂, wherein R' is selected from the group consisting of hydrogen, C₁-C₁₂-alkyl group, CN, C₂-C₆ alkenyl, aryl, cycloalkyl, aralkyl, heteroaryl, each of which is optionally substituted by one or more groups independently selected from the group consisting of -R", -X', -OR", -SR", -NR"₂, -SiR'₃, -COOR',-(C-O)R", -CN and -CONR"₂, wherein R" is hydrogen or a C₁-C₁₂-alkyl group and X' is F, Cl, Br, or I. Preferably, R³ and R⁴ are H and R¹ is methyl. R² often is an optionally substituted methyl or ethyl group. Preferably, R² is methyl, which is substituted by one, two or three halogen atoms, indepently selected from the group consisting of Cl, F and Br. More preferably, R² is selected from the group consisting of CF₃, CF₂Cl, CFHCl, CFH₂, CCl₂F, CH₂Cl, CF₂Br, CFHBr, CCl₂Br, CH₂Br and CBr₃. Even more preferably, R² is CF₃. In a preferred embodiment in relation with compound (V), R² is CF₃, R³ and R⁴ are H and R¹ is methyl.

The invention concerns further a process for the manufacture of the compound of formula (V), wherein a compound of formula (I) is reacted with a compound of formula (VI) 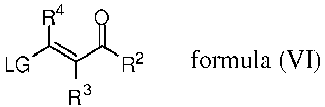 wherein LG is a leaving group selected from the group consisting of OR⁶, NR⁶₂ and SR⁶, wherein R⁶ is an optionally substituted C₁-C₄-alkyl group. Preferably, LG is OR⁶, wherein R⁶ is methyl or ethyl, preferably ethyl. R², R³ and R⁴ are independently selected from the group consisting of R', X', OR', SR', CN, C(O)R', COOR', C(O)NR'₂, wherein R' is selected from the group consisting of hydrogen, C₁-C₁₂-alkyl group, CN, C₂-C₆ alkenyl, aryl, cycloalkyl, aralkyl, heteroaryl, each of which is optionally substituted by one or more groups independently selected from the group consisting of -R", -X', -OR", -SR", - NR"₂, -SiR'₃, -COOR', -(C-O)R", -CN and -CONR"₂, wherein R" is hydrogen or a C₁-C₁₂-alkyl group and X' is F, Cl, Br, or I. R² often is an optionally substituted methyl or optionally substituted ethyl group. Preferably, R² is methyl, which is substituted by one, two or three halogen atoms, indepently selected from the group consisting of Cl, F and Br. More preferably, R² is selected from the group consisting of CF₃, CF₂Cl, CFHCl, CFH₂, CCl₂F, CH₂Cl, CF₂Br, CFHBr, CCl₂Br, CH₂Br and CBr₃. Even more preferably, R² is CF₃. In a most preferred embodiment, R² is CF₃, R³ and R⁴ are H and LG is OC₂H₅.

The invention further concerns a process for the manufacture of a compound of formula (VII) 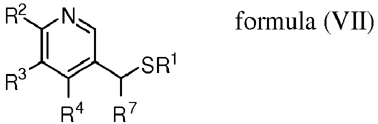 wherein R⁷ is an optionally substituted C₁-C₄ alkyl group, which comprises at least of the the processes above for the manufacture of the compound of formula (I) or (VII). R⁷ is optionally subsituted by one or more groups independently selected from the group consisting of R', X', OR', SR', CN, C(O)R', COOR', C(O)NR'₂, wherein R' is selected from the group consisting of hydrogen, C₁-C₁₂-alkyl group, CN, C₂-C₆ alkenyl, aryl, cycloalkyl, aralkyl, heteroaryl, each of which is optionally substituted by one or more groups independently selected from the group consisting of -R", -X', -OR", -SR", - NR"₂, -SiR'₃, -COOR', -(C-O)R", -CN and -CONR"₂, wherein R" is hydrogen or a C₁-C₁₂-alkyl group and X' is F, Cl, Br, or I. Preferably, R⁷ is CH₃. In a preferred embodiment in relation with compound (VII), R² is CF₃, R³ and R⁴ are H and R¹ and R⁷ are methyl.The process for the manufacture of compound (VII) can comprise one or more of the steps selected from the group consisting of elimination of water, which is preferably acid catalyzed, and nucleophilic alkylation, for example Grignard reaction.

The compound of formula (I) can also be produced by transesterification of R¹SH with alkylmalonate, wherein the process further comprises a step of amination, for example by treatment with ammonia or ammonia derivative and/or a base, or NH₄OH. According to one aspect of such a process, CH₃SH is transesterified with ethylmalonate to obtain (I) wherein R¹ is CH₃.

The invention further concerns a process for the manufacture of a agrochemical or pharmaceutical compound, which comprises at least one of the processes for the manufacture of compound (I), (VII) or (V). In one aspect, the process for the manufacture of a agrochemical compound which comprises at least one of the processes for the manufacture of compound (I), (VII) or (V) is a process for the manufacture of [Methyl(oxo){1-[6-trifluoromethyl)-3-pyridyl]ethyl}-λ⁶-sulfanylidene cyanamide.

The compounds of formula (I) are useful in the manufacture of agrochemically or pharmaceutically active substances. The processes according to the present invention often display a higher efficiency in respect to number of steps, yield, selectivity and waste.

Should the disclosure of any patents, patent applications, and publications which are incorporated herein by reference conflict with the description of the present application to the extent that it may render a term unclear, the present description shall take precedence.

Where in the present invention formulae contain stereogenic centres, the centre is intended to denote the (R) configuration, the (S) configuration a mixture of both configurations.

The following examples are intended to further explain the invention without limiting it.

### Examples

Malonic acid dichloride and NaSCH₃ are obtained from commercial sources. 4-ethoxy-1,1,1-trifluorobut-3-en-2-one (ETFBO) is obtained by the process described in EP1644306B1, Example 2.

### Example 1 : Manufacture of S-methyl 3-amino-3-oxopropanethioate

Malonic acid dichloride is reacted with NaSCH₃. The reaction mixture is treated with aq. HCl, and extracted with diethylether. The mixture is distilled to obtain 3-(methylthio)-3-oxopropanoic acid. 3-(methylthio)-3-oxopropanoic acid is reacted with (COCl)₂ in dimethylformamide and dichloromethane, followed by treatment with ammonia in dioxane to obtain S-methyl 3-amino-3-oxopropanethioate.

### Example 2 : S-methyl 2-oxo-6-(trifluoromethyl)-1,2-dihydropyridine-3-carbothioate

In a sealable vessel, 300 mg S-methyl 3-amino-3-oxopropanethioate (TAM) (2.25 mmol, 1 eq) were dissolved in 3 mL tetrahydrofurane (THF). Triethylamine (467 µL, 341 mg, 3.38 mmol, 1.5 eq) was added, and the mixture was stirred for 5 minutes. Then 311 µL ETFBO (2, 379 mg, 2.25 mmol, 1 eq) was added. The vessel was sealed, and the mixture was stirred at room temperature. After 3h GC analysis showed 85% product. Stirring was continued overnight. The solvent was removed in vacuo, giving 702 mg crude product (quant) as a brown liquid of 96% purity (GC). Column chromatography on silica led to the retention of the majority of crude product, yielding 153 mg product as an off-white solid. GC tr = 13.12 min GC/MS tr = 11.93 min (237 [M+], 190 (100), 162, 142, 114, 107). HPLC/ESI tr = 4.59 min (238 [M+H], 260 [M+Na], 301 [M+Na+MeCN], 497 [2M+Na]) 1H-NMR (90 MHz, DMSO-d6) δ (ppm) = 8.47 (d, J ≈ 7 Hz, 1H), 7.56 (d, J ≈ 7 Hz, 1H), 2.47 (s, 3H).

## Claims

1. Compound of the formula (I) 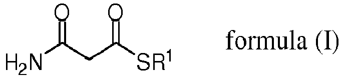 wherein R¹ is selected from the group consisting of H and optionally substituted C₁-C₄-alkyl.

2. Compound according to claim 1, wherein R¹ is methyl or ethyl.

3. Compound according to claim 2, wherein R¹ is methyl.

4. Compound according to anyone of claims 1 to 3, wherein R¹ is substituted with one or more substituents independently selected from the group consisting of R', X', OR', SR', CN, C(O)R', COOR', C(O)NR'₂, wherein R' is selected from the group consisting of hydrogen, C₁-C₁₂-alkyl group, CN, C₂-C₆ alkenyl, aryl, cycloalkyl, aralkyl, heteroaryl, each of which is optionally substituted by one or more groups independently selected from the group consisting of -R", -X', -OR", -SR", -NR"₂, -SiR'₃, -COOR', -(C-O)R", -CN and -CONR"₂, wherein R" is hydrogen or a C₁-C₁₂-alkyl group and X' is F, Cl, Br, or I.

5. Process for the manufacture of the compound of formula (I) as defined in anyone of claims 1 to 4, wherein the process comprises at least one of the following steps :
a) reacting malonic acid dichloride with a compound of formula MSR¹, wherein M is a monovalent alkali metal ion, preferably Na⁺, to obtain the compound of formula (III) 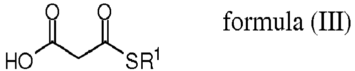
b) reacting the compound of formula (III) with a chlorinating agent to obtain a compound of formula (IV) 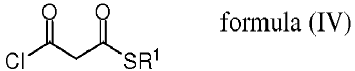
c) reacting a malonic ester of the formula (IX) R⁸OC(O)CH₂C(O)OR⁸ with a compound of the formula R¹SH, wherein R¹ is defined as in anyone of claims 1 to 4, to obtain a compound of the formula (VIII) 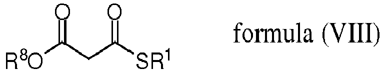 wherein R⁸ is a C₁-C₁₂-alkyl group, preferably methyl or ethyl.
d) reacting the compound of formula (IV) or (VIII) with ammonia or an ammonia derivative to obtain a compound of formula (I).

6. Use of the compound of formula (I) as defined in anyone of claims 1 to 4 for the manufacture of the compound of formula (V) 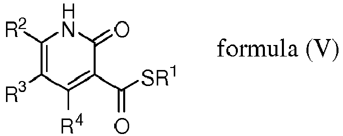 wherein R¹ is as defined above, and R², R³ and R⁴ are independently selected from the group consisting of R', X', OR', SR', CN, C(O)R', COOR', C(O)NR'₂, wherein R' is selected from the group consisting of hydrogen, C₁-C₁₂-alkyl group, CN, C₂-C₆ alkenyl, aryl, cycloalkyl, aralkyl, heteroaryl, each of which is optionally substituted by one or more groups independently selected from the group consisting of -R", -X', -OR", -SR", -NR"_{2,} -SiR'₃, -COOR',-(C-O)R", -CN and -CONR"₂, wherein R" is hydrogen or a C₁-C₁₂-alkyl group and X' is F, Cl, Br, or I.

7. Process for the manufacture of the compound of formula (V) as defined in claim 6, wherein a compound of formula (I) as defined in anyone of claims 1 to 4 is reacted with a compound of formula (VI) 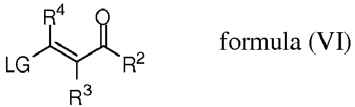 wherein LG is a leaving group selected from the group consisting of OR⁶, NR⁶₂ and SR⁶, wherein R⁶ is an optionally substituted C₁-C₄-alkyl group and R², R³ and R⁵ are defined as in claim 6.

8. Process according to claim 7, wherein LG is OR⁶, wherein R⁶ is methyl or ethyl, preferably ethyl.

9. Process according to claim 7 or 8, wherein R² is an optionally substituted methyl or optionally substituted ethyl group.

10. Process according to claim 9, wherein R² is methyl, which is substituted by one, two or three halogen atoms, independently selected from the group consisting of Cl, F and Br.

11. Process according to claim 10, wherein R² is selected from the group consisting of CF₃, CF₂Cl, CFHCl, CFH₂, CCl₂F, CH₂Cl, CF₂Br, CFHBr, CCl₂Br, CH₂Br and CBr₃, preferably wherein R² is CF₃.

12. Process for the manufacture of a compound of formula (VII) 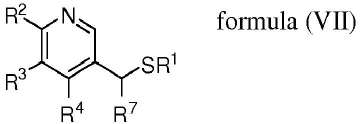 wherein R⁷ is an optionally substituted C₁-C₄ alkyl group, and R¹, R², R³ and R⁴ are as defined in anyone of claims 1 to 4, 6 or 9 to 11, which comprises the process according to anyone of claims 7 to 11.

13. Process according to claim 12, wherein R⁷ is methyl.

14. Process for the manufacture of an agrochemical or pharmaceutical compound, which comprises the process according to anyone of claims 7 to 13.
